(19) Europäisches Patentamt
European Patent Office
Office européen des brevets

(11) Numéro de publication: **0 129 461**
**B1**

(12) # FASCICULE DE BREVET EUROPEEN

(45) Date de publication du fascicule du brevet: **28.01.87**

(51) Int. Cl.[4]: **C 07 K 5/06**, A 61 K 37/02

(21) Numéro de dépôt: **84401145.2**

(22) Date de dépôt: **05.06.84**

(54) **Dérivés d'acides isoindoledicarboxyliques, leur préparation et compositions pharmaceutiques les contenant.**

(30) Priorité: **06.06.83 FR 8309326**

(43) Date de publication de la demande:
**27.12.84 Bulletin 84/52**

(45) Mention de la délivrance du brevet:
**28.01.87 Bulletin 87/5**

(84) Etats contractants désignés:
**AT BE CH DE FR GB IT LI LU NL SE**

(56) Documents cités:
**EP - A - 0 031 741**
**EP - A - 0 046 953**
**EP - A - 0 050 800**
**EP - A - 0 051 301**
**EP - A - 0 058 567**
**EP - A - 0 081 094**

(73) Titulaire: **ADIR, 22, rue Garnier,**
***F-92200 Neuilly-sur-Seine* (FR)**

(72) Inventeur: **Vincent, Michel, 8 allée du Prunier Hardy,**
**F-92220 Bagneux (FR)**
Inventeur: **Remond, Georges, 9 avenue des Etats Unis,**
**F-78000 Versailles (FR)**
Inventeur: **Laubie, Michel, 35 avenue Foch,**
**F-92420 Vaucresson (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention a pour objet des dérivés d'acides isoindole-dicarboxyliques substitués, leur préparation et les compositions pharmaceutiques les contenant.

Différents aminoacides possédant une action inhibitrice sur les enzymes comme les carboxypolypeptidases responsables de la transformation de l'Angiotensine I en Angiotensine II ou sur les enkephalines sont déjà connus. La demande de brevet EP 31 741 décrit certains alcools, acides, thiols, nitriles ou amines primaires dérivés des acides azabicycloalcane carboxyliques. D'autres aminoacides ayant une structure indolique, isoquinoléique ou azaalkanoylindolique sont décrits dans les demandes de brevet EP 46 953 et EP 51 301. Des dipeptides mono ou bicycliques sont aussi déjà connus (Demande de brevet EP 81 094). D'une manière surprenante très peu d'amino acides comportant une structure isoindolique ou perhydroisoindolique et possédant une activité inhibitrice des enzymes de conversion sont connus dans la littérature. Les seuls produits cités sont des perhydroisoindoles (Demande de brevet EP 58 567) ou des isoindoles (Demande de bevet EP 50 800) comportent à la chaîne latérale un reste aromatique. La demanderesse a maintenant découvert que certains dérivés d'acides isoindole carboxylique ayant une chaîne latérale uniquement aliphatique et ne comportant qu'une seule fonction aminée secondaire, ont une activité très puissante vis-à-vis de l'angiontensine convertase.

Spécifiquement, l'invention concerne les composés répondant à la formule générale:

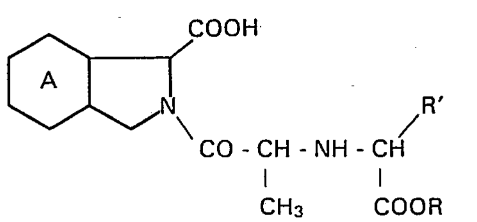

I

dans laquelle:

le cycle A est saturé ou bien benzénique,

R représente un atome d'hydrogène ou un groupe alkyle inférieur de 1 à 4 atomes de carbone, et

R' représente un radical alkyle linéaire de 1 à 6 atomes de carbone, un radical mono- ou dicycloalkylalkyle de 4 à 7 atomes de carbone,

sous forme racémique ou d'isomères optiques,

ainsi que leurs sels obtenus avec une base minérale ou organique thérapeutiquement acceptable, ou leurs sels d'addition obtenus avec un acide minéral ou organique thérapeutiquement acceptable.

Les composés de formule (I) comportent 3 atomes de carbone asymétrique (éventuellement 4 lorsque R = méthyl-2 propyle) dont 2 sur la chaîne latérale, le 3ème étant le carbone du cycle qui porte le groupe carboxy.

Lorsque A est saturé, le noyau perhydroisoindole correspondant n'existe pratiquement que sous la forme cis.

Les composés racémiques peuvent être dédoublés en leurs mélanges de diastéréoisomères ou d'épimères, ou dédoublés en leurs énantiomères de manière connue. Ces divers isomères font partie de l'invention de même que les composés racémiques. Toutefois, les isomères (S) sont plus actifs et par conséquent préférés.

Les composés selon l'invention ainsi que leurs sels possèdent des propriétés pharmacologiques intéressantes. Ils exercent notamment une activité inhibitrice sur certaines enzymes, comme les carboxypolypeptidases, les enképhalinases ou la kininase II. Ils inhibent notamment la transformation du décapeptide angiotensine I en l'octapeptide angiotensine II, responsable dans certains cas de l'hypertension artérielle, en agissant sur l'enzyme de conversion.

L'emploi en thérapeutique de ces composés permet donc de réduire ou même supprimer l'activité de ces enzymes responsables de la maladie hypertensive ou de l'insuffisance cardiaque. L'action sur la kininase II a pour résultat une augmentation de la bradykinine circulante et également une baisse de la tension artérielle.

L'invention s'étend aussi aux compositions pharmaceutiques renfermant comme principe actif au moins un composé de formule générale I ou un de ses sels d'addition, avec une base ou un acide minéral ou organique, en association avec un excipient inerte, non toxique, pharmaceutiquement acceptable.

En vue de l'emploi en thérapeutique, les composés de formule générale I ou leurs sels sont présentés sous des formes pharmaceutiques convenant pour l'administration par voie intraveineuse ou orale. Les compositions pharmaceutiques selon l'invention renferment, outre le principe actif, un ou plusieurs excipients inertes, non-toxiques convenant pour l'usage pharmaceutique et/ou un agent liant, un agent aromatisant, un agent de délitement, un agent édulcorant, un agent lubrifiant ou bien encore un véhicule liquide adapté à l'administration par voie intraveineuse.

Les compositions pharmaceutiques selon l'invention peuvent en outre contenir un autre principe actif d'action synergique ou complémentaire.

Parmi ces derniers principes actifs, on pourra citer un diurétique et, notamment, un saliurétique, comme par exemple un thiazide, un dihydrothiazide, un chlorosulfamide, un acide dihydrobenzofuranne 2-carboxylique ou un dérivé de l'acide phénoxy acétique. Des exemples de tels composés sont la N-(3'-chloro 4'-sulfamoyl benzamido) 2-méthyl indoline, l'acide éthacrynique, le furosémide.

La posologie utile peut varier largement en fonction de l'âge, du poids du patient, de la sévérité de l'indication thérapeutique ainsi que la voie d'administration. La voie d'administration préférée est la voie buccale mais la voie intraveineuse est également parfaitement appropriée au traitement de l'hypertension. D'une manière générale, la posologie unitaire s'échelonnera de préférence entre 1 et 100 mg.

Les composés de l'invention peuvent être préparés par condensation d'un dérivé de carboxy-iso-indole de formule:

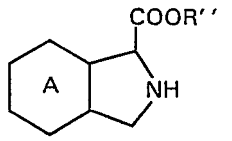

II

dans laquelle A a la même signification dans la formule I et,

R'' représente un groupe alkyle de 1 à 6 atomes de carbone, de préférence un groupe tertiobutyle, ou un de ses sels d'addition,

avec un dérivé fonctionnel d'un amino-acide substitué de formule

```
HOCO-CH-NH-CH-R'          III
     |     |
    CH3   COOR
```

dans laquelle R et R' ont la même signification que dans la formule I et l'atome d'azote est protégé par un radical habituel de protection de la synthèse des peptides, par exemple benzyloxycarbonyle ou tert.butoxycarbonyle, et le composé intermédiaire obtenu est soumis aux procédés de déprotection habituels tels que par exemple saponification totale ou partielle et/ou hydrogénolyse, et est ainsi transformé en un composé de formule I.

Le dérivé fonctionnel de l'amino-acide (III) peut être préparé in situ avec un agent tel que le dicyclohexylcarbodiimide seul ou en présence de hydroxy-1 benztriazole dans un solvant aprotique, par exemple le diméthylformamide.

Les composés de formule II sont décrits dans la littérature (G. CIGNARELLA et coll., Gazz. Chim. Ital. 106, 65-75, et demande de brevet européen N° 31 741).

Certains amino-acides de formule III ($R = C_2H_5$) sont connus [M. VINCENT et coll., Tetr. Lett. 23 (16), 1677-1680, 1982] ou peuvent être synthétisés de manière semblable.

Les exemples suivants illustrent l'invention.

*Exemple I*

*[N-⟨(S)-éthoxycarbonyl-1 butyl⟩-(S) alanyl]-2 (S)-carboxy-1 isoindoline, sel de tert.butylamine*

*Stade A:* Chlorhydrate de (R,S) tert.butyloxycarbonyl-1 isoindoline.

Par réaction de 30 g de chlorhydrate de (R,S) méthoxycarbonyl-1 isoindoline (préparée selon G. CIGNARELLA et coll., Gazz. Chim. Ital. 106, 65-75, 1976) avec 31 $cm^3$ de chlorure de benzyloxycarbonyle selon la méthode décrite par J.P. GREENSTEIN et M. WINITZ dans «Chemistry of the Amino Acids» vol. 2, page 887, on obtient 47,2 g de N-benzyloxycarbonyl (R,S) méthoxycarbonyl-1 isoindoline qui donnent par saponification 39,4 g de N-benzyloxycarbonyl (R,S) carboxy-1 isoindoline. Cet acide est traité en totalité dans 200 $cm^3$ de chlorure de méthylène par 100 mg d'isobutylène en présence d'un $cm^3$ d'acide sulfurique selon la méthode décrite par G.W. ANDERSON, F.M. CALLAHAN, J. Am. Chem. Soc. 82, 3359, (1960). On obtient ainsi 43,5 g de N-benzyloxycarbonyl (R,S) tert.butyloxycarbonyl-1 isoindoline qui après hydrogénolyse (éthanol, Pd/C 10%; 2,5 kg/$cm^2$) et précipitation de chlorhydrate dans l'acétate d'éthyle par la quantité stoechiométrique d'acide chlorhydrique donnent 27,6 g de chlorhydrate de (R,S) tert.butyloxycarbonyl-1 isoindoline.

F = 152°C (Décomposition).

Analyse: $C_{13}H_{17}NO_2$, HCl

| | | | | |
|---|---|---|---|---|
| Calculé: | C 61,05 | H 7,09 | N 5,48 | Cl 13,86 |
| Trouvé: | C 60,82 | H 7,05 | N 5,42 | Cl 13,29 |

Spectre IR: $NH_2^+$: 2300-2800 $cm^{-1}$ CO (ester) 1730 $cm^{-1}$

*Stade B:* [N-⟨(S)-éthoxycarbonyl-1 butyl⟩-(S) alanyl]-2 (S) tert.butyloxycarbonyl-1 isoindoline.

Dans un ballon muni d'une agitation et d'une garde à chlorure de calcium, on introduit 6 g de chlorhydrate de (R,S) tert.butyloxycarbonyl-1 isoindoline (obtenus au stade A), 40 $cm^3$ de diméthylformamide anhydre et 3,4 $cm^3$ de triéthylamine. On ajoute successivement 4,6 g de N-[(S) éthoxycarbonyl-1 butyl]-(S) alanine [preparée selon M. VINCENT et coll., Tetrahedron Letters, Vol. 23, N° 1b, p. 1677-1680 (1982)] en solution dans 50 $cm^3$ de diméthylformamide anhydre, une solution de 3,05 g de N-hydroxybenzotriazole dans 80 $cm^3$ de diméthylformamide anhydre puis une solution de 4,82 g de dicyclohexylcarbodiimide dans 40 $cm^3$ de chlorure de méthylène. On agite 24 heures à température ambiante, filtre la dicyclohexylurée formée et évapore le filtrat sous vide. Le résidu est dissous dans 200 $cm^3$ d'acétate d'éthyle, on lave cette solution par 2 fois 50 $cm^3$ d'une solution saturée de bicarbonate de sodium puis 3 fois par 50 $cm^3$ d'une solution saturée de chlorure de sodium. On sèche sur sulfate de calcium et évapore à sec. Le résidu (7,3 g) est chromatographié sur gel de silice (éluant acétate d'éthyle). On sépare ainsi le N-[(S)-éthoxycarbonyl-1 butyl]-(S) alanyl -2 (S)-tert.butyloxycarbonyl-1 isoindoline (Rf = 0,45-2,5 g) du N-[(S)-éthoxycarbonyl-1 butyl)-(S) alanyl] -2 (R)-tert.butyloxycarbonyl-1 isoindoline (Rf = 0,30-1,7 g).

*Stade C:* [N-⟨(S) éthoxycarbonyl-1 butyl⟩-(S) alanyl]-2 (S) carboxy-1 isoindoline, sel de tert.-butylamine.

On dissout 1,2 g de [N-⟨(S)-éthoxycarbonyl-1 butyl⟩-(S) alanyl]-2 (S)-tert.butyloxycarbonyl-1 isoindoline (obtenu au stade précédent) dans 100 $cm^3$ d'acétate d'éthyle chlorhydrique 4N. On abandonne la solution 18 heures à température ambiante puis évapore à sec. Le résidu est repris par 100 $cm^3$ d'eau et extrait une fois par 100 $cm^3$ d'éther éthylique. La phase aqueuse est amenée à pH = 4 par de la soude 1N puis évaporée à sec. On reprend par 50 $cm^3$ d'éthanol anhydre, filtre le chlorure de sodium et évapore à sec le filtrat. Le résidu est repris par 50 $cm^3$ d'éther éthylique anhydre. On filtre sur talc et ajoute 1,5 $cm^3$ de tert.butylamine au filtrat, le sel précipite immédiatement; on filtre et lave 2 fois par 20 $cm^3$ d'éther éthylique anhydre. On obtient ainsi 0,8 g de produit.

Analyse: $C_{19}H_{26}N_2O_5$, $C_4H_{11}N$

| | | | |
|---|---|---|---|
| Calculé: | C 63,42 | H 8,56 | N 9,65 |
| Trouvé: | C 63,05 | H 8,49 | N 9,47 |

Spectre IR: bande $NH_3^+$: 2100-2800 $cm^{-1}$
$COO^-$: 1580 $cm^{-1}$
CO (ester): 1725-1740 $cm^{-1}$
CO (amide): 1645 $cm^{-1}$

*Exemple II*

*[N-⟨(S)-éthoxycarbonyl-1 butyl⟩-(S) alanyl]-2 (R)-carboxy-1 isoindoline*

On dissout 1 g de [N-⟨(S)-éthoxycarbonyl-1 butyl⟩-(S) alanyl]-2 (R)-tert.butyloxycarbonyl-1 isoindoline (obtenu au stade B de l'exemple I) dans 80 $cm^3$ d'acétate d'éthyle chlorhydrique 4N et abandonne la solution 18 heures à température ambiante. On évapore à sec, reprend par 100 $cm^3$ d'eau et extrait par 100 $cm^3$ d'éther éthylique. La phase aqueuse est amenée à pH = 4 par de la soude 1N puis évaporée à sec. Le résidu est repris par 50 $cm^3$ d'éthanol anhydre, on filtre le chlorure de sodium et évapore le filtrat. Le résidu est repris par 50 $cm^3$ de chlorure de méthylène, on filtre sur talc, amène à sec, redissout dans 25 $cm^3$ d'eau et lyophilise, on obtient ainsi 0,42 g de produit.

Analyse: $C_{19}H_{26}N_2O_5$

| | | | |
|---|---|---|---|
| Calculé: | C 62,97 | H 7,23 | N 7,73 |
| Trouvé: | C 62,93 | H 7,03 | N 7,56 |

Spectre IR: bandes OH et NH 2000-3700 $cm^{-1}$
CO: 1655 et 1735 $cm^{-1}$

*Exemple III*

*[N-⟨(S)-éthoxycarbonyl-1 butyl⟩-(S) alanyl]-2 (S)-carboxy-1 perhydroisoindole, maléate*

*Stade A:* Chlorhydrate de (R,S) tert.butyloxycarbonyl-1 perhydroisoindole.

Dans un autoclave d'un litre, on introduit 300 g de tert.butanol fondu, 20 g de chlorhydrate de (R,S) tert.butyloxycarbonyl-1 isoindoline (Stade A exemple I) et 4 g de Rhodium sur alumine. On hydrogène 24 heures à 40°C sous une pression de 60 kg/$cm^2$. On filtre, lave le catalyseur par 50 $cm^3$ d'éthanol anhydre et évapore à sec le filtrat. Le résidu est repris par 50 $cm^3$ d'acétone anhydre, on filtre le précité et le lave deux fois par 30 $cm^3$ d'acétone anhydre, on obtient ainsi 7,7 g de chlorhydrate de (R,S) tert.-butyl-oxycarbonyl-1 perhydroisoindole. F = 171°C.

*Stade B:* [N-⟨(S)-éthoxycarbonyl-1 butyl⟩-(S) alanyl]-2 (S) tert.butyloxycarbonyl-1 perhydroisoindole.

En utilisant le mode opératoire décrit dans l'exemple I stade B, on obtient à partir de 6,2 g de chlorhydrate de tert.butyloxycarbonyl-1 perhydroisoindole (obtenu au stade A) et après chromatographie sur gel de silice (éluant acétate d'éthyle 3 g de [N-⟨(S)-éthoxycarbonyl-1 butyl⟩-(S) alanyl]-2 (S)- tert.butyloxycarbonyl-1 perhydroisoindole (Rf = 0,42) et 2,1 g de [N-⟨(S)-éthoxy-carbonyl-1 butyl⟩-(S) alanyl]-2 (R)-tert.butyloxycarbonyl-1 perhydroisoindole (Rf = 0,30).

*Stade C:* Maléate de [N-⟨(S)-éthoxycarbonyl-1 butyl⟩-(S) alanyl]-2 (S)-carboxy-1 perhydroisoindole.

On dissout 1,5 g de [N-⟨(S)-éthoxycarbonyl-1 butyl⟩-(S) alanyl]-2 (S)-tert.butyloxycarbonyl-1 perhydroisoindole obtenu au stade B dans $cm^3$ d'acétate d'éthyle chlorhydrique 4N et abandonne la solution 24 heures à température ambiante. On évapore à sec et reprend par 100 $cm^3$ d'eau et 100 $cm^3$ d'éther éthylique. La phase aqueuse est amenée à pH = 4 par de la soude 1N puis saturée par du chlorure de sodium. On extrait 4 fois par 100 $cm^3$ de chlorure de méthylène, les phases organiques sont jointes et lavées 1 fois par 50 $cm^3$ d'une solution saturée de chlorure de sodium. On sèche sur sulfate de calcium et évapore à sec. On reprend par 100 $cm^3$ d'éther éthylique, filtre sur talc un trouble et ajoute au filtrat une solution de 2 g d'acide maléique dans 100 $cm^3$ d'éther éthylique; le maléate (hygroscopique) précipite. On décante l'éther et lave le résidu 2 fois par 100 $cm^3$ d'éther éthylique. On redissout dans 25 $cm^3$ d'eau et lyophilise on obtient ainsi 1 g de produit.

Analyse: $C_{19}H_{32}N_2O_5$, $C_4H_4O_4$

| | | | |
|---|---|---|---|
| Calculé: | C 57,01 | H 7,49 | N 5,78 |
| Trouvé: | C 56,84 | H 7,20 | N 5,80 |

Spectre IR: bandes OH et $NH_2^+$: 2200-3600 $cm^{-1}$
CO (amide et ester) 1740 $cm^{-1}$
$COO^-$ 1620-1660 $cm^{-1}$

*Exemple IV:*

*[N-⟨(S)-éthoxycarbonyl-1 butyl⟩-(S) alanyl]-2 (R)-carboxy-1 perhydroisoindole*

En remplacant dans le procédé décrit pour l'exemple II le [N-⟨(S)-éthoxycarbonyl-1 butyl⟩-(S) alanyl]-2 (R) tert.butylcarbonyl-1 isoindoline par 1,1 g de [N-⟨(S)-éthoxycarbonyl-1 butyl⟩-(S) alanyl]-2 (R) tert.butyloxycarbonyl-1 perhydroisoindole obtenu dans l'exemple III stade B, on obtient après lyophilisation 0,7 g de [N-⟨(S)-éthoxycarbonyl-1 butyl⟩-(S) alanyl]-2 (R) carboxy-1 perhydroisoindole.

Analyse: $C_{19}H_{32}N_2O_5$

| | | | |
|---|---|---|---|
| Calculé: | C 61,93 | H 8,75 | N 7,60 |
| Trouvé: | C 61,79 | H 8,54 | N 7,60 |

Spectre IR: bande OH et $NH_2^+$: 2300-3600 $cm^{-1}$
CO ester 1730 $cm^{-1}$
$COO^-$ et amide 1600-1650 $cm^{-1}$

D'une manière identique l'on prépare les composés suivants:

[N-⟨(S)-éthoxycarbonyl-1 propyl⟩-(S) alanyl]-2 (S) carboxy-1 isoindoline
[N-⟨(S)-éthoxycarbonyl-1 propyl⟩-(S) alanyl]-2 (S) carboxy-1 perhydroisoindole
[N-⟨(S)-éthoxycarbonyl-1 pentyl⟩-(S) alanyl]-2 (S) carboxy-1 perhydroisoindole
[N-⟨(S)-éthoxycarbonyl-1 pentyl⟩-(S) alanyl]-2 (S) carboxy-1 isoindoline
[N-⟨(S)-éthoxycarbonyl-1 cyclopropyl-2 éthyl⟩-(S) alanyl]-2 (S) carboxy-1 isoindoline
[N-⟨(S)-éthoxycarbonyl-1 cyclopropyl-2 éthyl⟩-(S) alanyl]-2 (S) carboxy-1 perhydroisoindole
[N-⟨(S)-éthoxycarbonyl-1 dicyclopropyl-2, 2 éthyl⟩-(S) alanyl]-2 (S) carboxy-1 isoindoline
[N-⟨(S)-éthoxycarbonyl-1 dicyclopropyl-2, 2 éthyl)-(S) alanyl]-2 (S) carboxy-1 perhydroisoindole

Les composés de l'invention ont été soumis à une étude pharmacologique chez le rat anesthésié selon le protocole décrit par D.M. GROSS et coll. [J. Pharmacol. and Exp. Ther. 216, 552-557, 1981)].

La dose I.V. capable d'inhiber 50% de l'activité de l'angiotensine convertase présente dans la circulation ($DE_{50}$) a été déterminée. Les résultats sont les suivants:

| COMPOSE DE L'EXEMPLE | $DE_{50}$ (mg/kg) |
|---|---|
| I | 0,022 |
| II | 0,170 |
| III | 0,020 |
| IV | >300 |

EXEMPLE DE FORMULATION

| | |
|---|---|
| [N-<(S)-éthoxycarbonyl-1 butyl>-(S) alanyl]-2 (S) carboxy-1 perhydroisoindole (maléate) | 10 mg |
| amidon de blé | 120 mg |
| amidon de maïs | 115 mg |
| caséine formolée | 20 mg |
| stéarate de magnésium | 15 mg |
| talc | 20 mg |

pour 1 comprimé.

**Revendications pour les Etats contractants:** BE, CH, DE, FR, GB, IT, LI, LU, NL, SE

1. Composé répondant à la formule générale I:

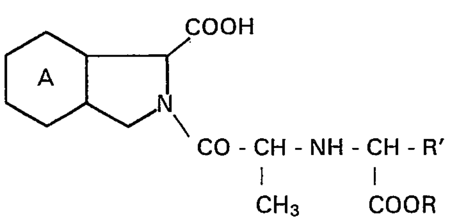

I

dans laquelle:

le cycle A est saturé ou bien benzénique,

R représente un atome d'hydrogène ou un groupe alkyle inférieur de 1 à 4 atomes de carbone,

R' représente un radical alkyle linéaire de 1 à 6 atomes de carbone, un radical mono- ou dicycloalkylalkyle de 4 à 7 atomes de carbone,

sous leur forme racémique ou d'isomères optiques, et leurs sels obtenus avec une base minérale ou organique thérapeutiquement compatible, ou leurs sels d'addition obtenus avec un acide minéral ou organique pharmaceutiquement compatible.

2. La [N-<(S)-éthoxycarbonyl-1 butyl>-(S) alanyl]-2 (R,S) carboxy-1 oerhydroisoindole, son isomère (S), ainsi que leurs sels.

3. La [N-<(S)-éthoxycarbonyl-1 butyl>-(S) alanyl]-2 (R,S) carboxy-1 isoindoline, son isomère (S), ainsi que leurs sels.

4. Composition pharmaceutique contenant comme principe actif un composé selon l'une quelconque des revendications 1 à 3 ainsi qu'un excipient pharmaceutiquement acceptable.

5. Procédé de préparation des composés selon la revendication 1, procédé caractérisé en ce que l'on effectue la condensation d'un dérivé de carboxyisoindole de formule:

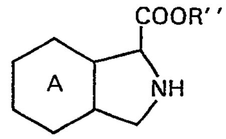

II

dans laquelle A a la même signification dans la formule I et,

R'' représente un groupe alkyle de 1 à 6 atomes de carbone, de préférence un groupe tertiobutyle, ou un de ses sels d'addition,

avec un dérivé fonctionnel d'un amino-acide substitué de formule III:

```
HOCO-CH-NH-CH-R'          III
     |     |
     CH3   COOR
```

dans laquelle R et R' ont la même signification que dans la formule I et l'atome d'azote est protégé par un radical habituel de protection de la synthèse des peptides, tel que benzyloxycarbonyle ou tert.butoxycarbonyle, et le composé intermédiaire obtenu est soumis aux procédés de déprotection habituels tels que par exemple saponification totale ou partielle et/ou hydrogénolyse, et est ainsi transformé en un composé de formule I.

**Revendication pour l'Etat contractant:** AT

1. Procédé de préparation de composés répondant à la formule générale I:

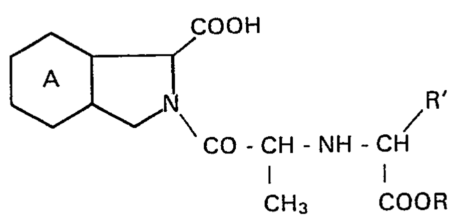

I

dans laquelle:

le cycle A est saturé ou bien benzénique,

R représente un atome d'hydrogène ou un groupe alkyle inférieur de 1 à 4 atomes de carbone,

R' représente un radical alkyle linéaire de 1 à 6 atomes de carbone, un radical mono- ou dicycloalkylalkyle de 4 à 7 atomes de carbone,

sous leur forme racémique ou d'isomères optiques, et de leurs sels obtenus avec une base minérale ou organique thérapeutiquement compatible, ou de leurs sels d'addition obtenus avec un acide minéral ou organique pharmaceutiquement compatible caractérisé en ce que l'on effectue la condensation d'un dérivé de carboxyisoindole de formule:

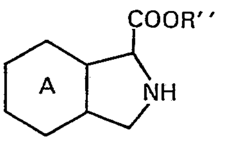

II

dans laquelle A a la même signification dans la formule I et,

R'' représente un groupe alkyle de 1 à 6 atomes de carbone, de préférence un groupe tertiobutyle, ou un de ses sels d'addition,

avec un dérivé fonctionnel d'un amino-acide substitué de formule III:

```
HOCO-CH-NH-CH-R'        III
     |     |
    CH3   COOR
```

dans laquelle R et R' ont la même signification que dans la formule I et l'atome d'azote est protégé par un radical habituel de protection de la synthèse des peptides, tel que benzyloxycarbonyle ou tert.-butoxycarbonyle, et le composé intermédiaire obtenu est soumis aux procédés de déprotection habituels tels que par exemple saponification totale ou partielle et/ou hydrogénolyse, et est ainsi transformé en un composé de formule I.

**Patentansprüche für die Vertragsstaaten:**
BE, CH, DE, FR, IT, LI, LU, NL, SE

1. Verbindungen der allgemeinen Formel I

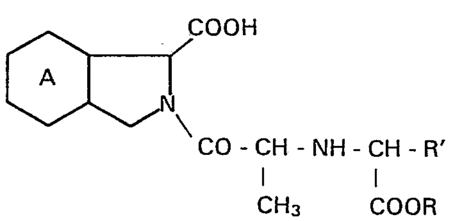

I

in der der Ring A gesättigt oder ein Benzolring ist und

R ein Wasserstoffatom oder eine niedrigmolekulare Alkylgruppe mit 1 bis 4 Kohlenstoffatomen und

R' eine geradkettige Alkylgruppe mit 1 bis 6 Kohlenstoffatomen oder eine Mono- oder Dicycloalkylalkylgruppe mit 4 bis 7 Kohlenstoffatomen bedeuten,

in Form der Racemate oder der optischen Isomeren und deren Salze mit anorganischen oder organischen, therapeutisch verträglichen Basen oder deren Additionssalze mit anorganischen oder organischen, pharmazeutisch verträglichen Säuren.

2. 2-[N-(S)-⟨1-(S)-ethoxycarbonyl-butyl⟩-alanyl]-1-(R,S)-carboxy-perhydroisoindol, dessen (S)-Isomeres sowie deren Salze.

3. 2-[N-(S)-⟨1-(S)-ethoxycarbonyl-butyl⟩-alanyl]-1-(R,S)-carboxy-isoindolin, dessen (S)-Isomeres und deren Salze.

4. Pharmazeutische Zubereitung enthaltend als Wirkstoff eine Verbindung nach einem der Ansprüche 1 bis 3 sowie ein pharmazeutisch annehmbares Trägermaterial.

5. Verfahren zur Herstellung der Verwendung nach Anspruch 1, dadurch gekennzeichnet, dass man ein Carboxyisoindol-Derivat der Formel II

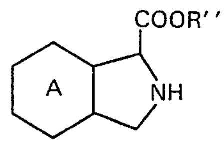

II

in der A die bezüglich der allgemeinen Formel I angegebenen Bedeutungen besitzt und

R'' eine Alkylgruppe mit 1 bis 6 Kohlenstoffatomen und vorzugsweise eine tert.-Butylgruppen bedeutet, oder ein Additionssalz davon

mit einem funktionellen Derivat einer substituierten Aminosäure der Formel III

```
HOCO-CH-NH-CH-R'        III
     |     |
    CH3   COOR
```

in der R und R' die bezüglich der allgemeinen Formel I angegebenen Bedeutungen besitzen, und dessen Stickstoffatom mit einer üblichen Schutzgruppe für die Peptidsynthese, wie eine Benzyloxycarbonylgruppe oder eine tert.-Butoxycarbonylgruppe, geschützt ist, kondensiert und das erhaltene Zwischenprodukt einer üblichen Abspaltung der Schutzgruppen, beispielsweise durch vollständige oder teilweise Verseifung und/oder Hydrogenolyse, unterwirft und in dieser Weise in die Verbindung der Formel I umwandelt.

**Patentanspruch für den Vertragsstaat:** AT

Verfahren zur Herstellung von Verbindungen der allgemeinen Formel I

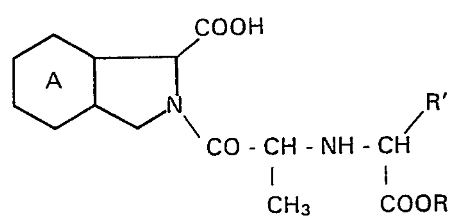

I

in der der Ring A gesättigt oder ein Benzolring ist und

R ein Wasserstoffatom oder eine niedrigmolekulare Alkylgruppe mit 1 bis 4 Kohlenstoffatomen und

R' eine geradkettige Alkylgruppe mit 1 bis 6 Kohlenstoffatomen oder eine Mono- oder Dicycloalkylalkylgruppe mit 4 bis 7 Kohlenstoffatomen bedeuten,

in Form der Racemate oder der optischen Isomeren, und ihrer Salze mit einer anorganischen oder organischen, pharmazeutisch verträglichen Base oder ihrer Additionssalze mit einer anorganischen und organischen, pharmazeutisch verträglichen Säure,

dadurch gekennzeichnet, dass man ein Carboxyisoindol-Derivat der Formel II

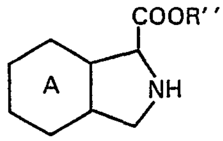

II

in der A die bezüglich der allgemeinen Formel I angegebenen Bedeutungen besitzt und

R'' eine Alkylgruppe mit 1 bis 6 Kohlenstoffatomen und vorzugsweise eine tert.-Butylgruppen bedeutet, oder eines seiner Additionssalze

mit einem funktionellen Derivat einer substituierten Aminosäure der Formel III

HOCO-CH-NH-CH-R' III<br>
| |<br>
$CH_3$ COOR

in der R und R' die bezüglich der allgemeinen Formel I angegebenen Bedeutungen besitzen und dessen Stickstoffatom durch eine üblichen Schutzgruppe für die Peptidsynthese, wie eine Benzyloxycarbonylgruppe oder eine tert.-Butoxycarbonylgruppe geschützt ist, kondensiert und das erhaltene Zwischenprodukt üblichen Verfahren zur Schutzgruppenabspaltung, beispielsweise durch vollständige oder teilweise Verseifung und/oder Hydrogenolyse unterwirft und in dieser Weise in die Verbindung der Formel I umwandelt.

**Claims for the contracting states:**
BE, CH, DE, FR, GB, IT, LI, LU, NL, SE

1. Compounds corresponding to the general formula I:

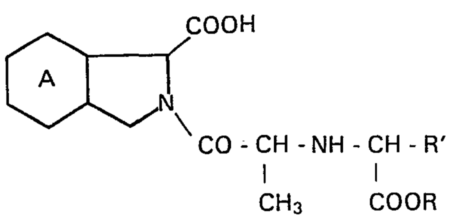

I

in which

the ring A is saturated or, alternatively, benzenic,

R represents a hydrogen atom or a lower alkyl group having from 1 to 4 carbon atoms,

R' represents a linear alkyl radical having from 1 to 6 carbon atoms or a mono- or di-cycloalkyl-alkyl radical having from 4 to 7 carbon atoms,

in their racemic form or in the form of optical isomers, and the salts thereof obtained with a therapeutically compatible mineral or organic base or the addition salts thereof obtained with a pharmaceutically compatible mineral or organic acid.

2. 2-[N-⟨1-(S)-ethoxycarbonylbutyl⟩-(S)-alanyl]-1-(R,S)-carboxyperhydroisoindole, its (S)-isomer, and also their salts.

3. 2-[N-⟨1-(S)-ethoxycarbonylbutyl⟩-(S)-alanyl]-1-(R,S)-carboxyisoindoline, its (S)-isomer, and also their salts.

4. Pharmaceutical composition containing a compound according to any one of claims 1 to 3 as active ingredient and also a pharmaceutically acceptable excipient.

5. Process for the preparation of the compounds according to claim 1, characterised in that a carboxyisoindole derivative of the formula:

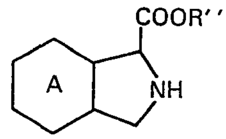

II

in which

A has the same meaning as in formula I and

R'' represents an alkyl group having from 1 to 6 carbon atoms, preferably a tert.-butyl group, or one of the addition salts thereof,

is condensed with a functional derivative of a substituted amino acid of the formula III:

HOCO-CH-NH-CH-R' III<br>
| |<br>
$CH_3$ COOR

in which

R and R' have the same meanings as in formula I and the nitrogen atom is protected by a radical customarily used for protection in the synthesis of peptides, such as benzyloxycarbonyl or tert.-butoxycarbonyl, and the intermediate compound obtained is subjected to customary deprotecting processes, such as, for example, total or partial saponification, and/or hydrogenolysis, and in this manner is converted into a compound of the formula I.

**Claim for the contracting state:** AT

Process for the preparation of compounds corresponding to the general formula I:

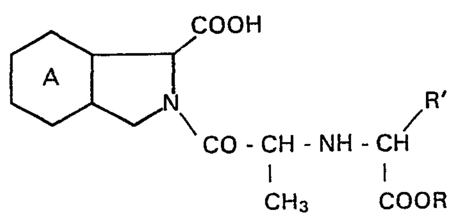

I

in which

the ring A is saturated or, alternatively, benzenic,

R represents a hydrogen atom or a lower alkyl group having from 1 to 4 carbon atoms,

R' represents a linear alkyl radical having from 1 to 6 carbon atoms or a mono- or di-cycloalkyl-alkyl radical having from 4 to 7 carbon atoms,

in their racemic form or in the form of optical isomers, and the salts thereof obtained with a therapeutically compatible mineral or organic base or the addition salts thereof obtained with a pharmaceutically compatible mineral or organic acid,

characterised in that a carboxyisoindole derivative of the formula:

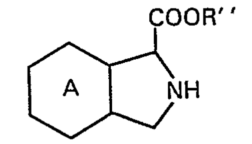

II

in which

A has the same meaning as in formula I and

R'' represents an alkyl group having from 1 to 6 carbon atoms, preferably a tert.-butyl group, or one of the addition salts thereof,

is condensed with a functional derivative of a substituted amino acid of the formula III:

HOCO-CH-NH-CH-R'     III
       |         |
     $CH_3$   COOR

in which R et R' have the same meanings as in formula I and the nitrogen atom is protected by a radical customarily used for protection in the synthesis of peptides, such as benzyloxycarbonyl or tert.-butoxycarbonyl, and the intermediate compound obtained is subjected to customary deprotecting processes, such as, for example, total or partial saponification, and/or hydrogenolysis, and in this manner is converted into a compound of the formula I.